# EUROPEAN PATENT APPLICATION

(11) **EP 4 039 326 A1**
(43) Date of publication of application: **10.08.2022**
(21) Application number: 20871598.7
(22) Date of filing: 24.09.2020
(51) Int. Cl.: A61P 1/00, A61P 1/04, A23L 33/12, A61K 31/202

(54) **COMPOSITION FOR IMPROVING INTESTINAL FUNCTION THROUGH EXPRESSION CONTROL OF AQUAPORIN 3, AND USE THEREOF**

(30) Priority: 01.10.2019 JP 2019181575
(71) Applicant: Suntory Holdings Limited, Osaka 530-8203 (JP)
(72) Inventor: MORITA, Satoshi, Soraku-gun, Kyoto 619-0284 (JP); KOMINAMI, Masaru, Soraku-gun, Kyoto 619-0284 (JP)
(74) Representative: Vossius & Partner Patentanwälte Rechtsanwälte mbB
(86) International application number: PCT/JP2020/035971
(87) International publication number: WO 2021/065659

(57) **Abstract**

The present invention aims to provide a composition for improving intestinal function through regulation of aquaporin 3 expression, use of the composition, a method of improving intestinal function through regulation of aquaporin 3 expression, and a method of alleviating intestinal discomfort through regulation of aquaporin 3 expression. The present invention relates to a composition for improving intestinal function through regulation of aquaporin 3 expression, containing dihomo-γ-linolenic acid as an active ingredient.

## Description

### TECHNICAL FIELD

The present invention relates to a composition for improving intestinal function through regulation of aquaporin 3 expression, and use of the composition. The present invention also relates to a method of improving intestinal function and a method of alleviating intestinal discomfort.

### BACKGROUND ART

Aquaporin (AQP) plays an important role in water transport in the human body. Aquaporin selectively allows water to permeate therethrough. Transport of water through aquaporin is controlled by the expression level of aquaporin. Aquaporin is expressed in various organs, and AQP has been known to have 13 types of AQPs (AQP0 to AQP12) so far (Non-Patent Literature 1).

AQP3 is the type that is mainly expressed particularly in the intestine. AQP3 is also involved in transport of low molecular weight compounds such as glycerol and urea in addition to water, and plays a role in control of the amount of water. Experiments using some drugs have revealed that decreasing the expression level of AQP3 in the intestine causes diarrhea and increasing the expression level of AQP3 in the intestine causes severe constipation (Non-Patent Literature 2). As described above, AQP3 in the intestine is a molecule that plays an important role in water transport in the intestine.

Further, AQP3 expression is known to be essential in maintenance of intestinal barrier (Non-Patent Literature 3). AQP3 in the intestine is a molecule that plays an important role also in maintenance of the intestinal barrier.

Meanwhile, dihomo-γ-linolenic acid (DGLA) is one of n-6 polyunsaturated fatty acids (PUFAs) and is contained in various foods including meat, eggs, and fish. DGLA has a long history of consumption, and is thus considered to be a very safe material. However, the amount of DGLA in various foods is generally very small, compared to arachidonic acid of the same n-6 PUFA or EPA or DHA of the same n-3 PUFA.

DGLA is known to be able to effectively suppress an increase in the number of mast cells. DGLA is known to be highly useful in various diseases deeply related to an increase in the number of mast cells, such as skin diseases, asthma, and rhinitis (Patent Literature 1).

DGLA is very useful in suppressing eosinophil infiltration, and is known to have a preventive or therapeutic effect against various diseases deeply related to eosinophil infiltration and an increase in the number of cells (e.g., skin diseases such as atopic dermatitis, eczema, and psoriasis; respiratory diseases such as bronchial asthma, chronic obstructive pulmonary disease (COPD), hypersensitivity pneumonitis, and eosinophilic pneumonitis; and gastrointestinal diseases such as eosinophilic gastroenteritis and ulcerative colitis) (Patent Literature 2).

However, the ability of DGLA to enhance the function of aquaporin has been unknown. In addition, the ability of DGLA to improve the intestinal function through regulation of aquaporin 3 expression has been unknown.

### CITATION LIST

### - Patent Literature

Patent Literature 1: JP 2006-306813 A
Patent Literature 2: JP 2006-306812 A

### - Non-Patent Literature

Non-Patent Literature 1: Toshiyuki Matsuzaki, The Medical Association of Nippon Medical School, 5, 118-124, 2009
Non-Patent Literature 2: Ikarashi N, Int. J. Mol. Sci., 17, E1172, 2016
Non-Patent Literature 3: Zhang W, FEBS Lett., 585, 3113-3119, 2011

### SUMMARY OF INVENTION

### - Technical Problem

The present invention aims to provide a composition for improving intestinal function through regulation of aquaporin 3 expression and use of the composition. The present invention also aims to provide a method of improving intestinal function through regulation of aquaporin 3 expression, and a method of alleviating intestinal discomfort through regulation of aquaporin 3 expression.

### - Solution to Problem

As a result of extensive studies to solve the above issues, the present inventors discovered that dihomo-γ-linolenic acid (DGLA) is very useful in improving the intestinal function through regulation of aquaporin 3 expression. The present invention was thus completed.

DGLA is contained in various foods including meat, eggs, and fish and has a long history of consumption, so that DGLA is considered to be a very safe material, as described above. The amount of DGLA in various foods is known to be generally very small, compared to the amount of arachidonic acid of the same n-6 PUFA or EPA or DHA of the same n-3 PUFA. As disclosed in Japanese Patent No. 3354581, the present applicant had invented a method of fermenting and producing DGLA lipids using a strain, and made it possible to mass produce triglyceride in which DGLA accounts for about 40% of its constituent fatty acids.

The present invention provides a composition for improving intestinal function through regulation of aquaporin 3 expression and use thereof, and relates to the following composition and the like.
(1) A composition for improving intestinal function through regulation of aquaporin 3 expression, containing: dihomo-γ-linolenic acid as an active ingredient.
(2) The composition for improving intestinal function according to (1) above, wherein the regulation of aquaporin 3 expression is inhibition of decrease in aquaporin 3 expression.
(3) The composition for improving intestinal function according to (2) above, wherein the inhibition of decrease in aquaporin 3 expression is inhibition of aging-induced decrease in aquaporin 3 expression.
(4) The composition for improving intestinal function according to any one of (1) to (3) above, wherein the composition for improving intestinal function is a food or beverage, a pharmaceutical product, or a quasi-pharmaceutical product.
(5) The composition for improving intestinal function according to any one of (1) to (4) above, wherein the composition is used for one or two or more purposes selected from the group consisting of alleviating intestinal discomfort, regulating the intestine, clearing the intestine, improving fecal conditions, improving bowel movement, improving bowel movement habit, improving intestinal conditions, enhancing intestinal barrier function, maintaining intestinal function, and improving decreased intestinal function.
(6) The composition for improving intestinal function according to any one of (1) to (5) above, wherein the composition is labeled as having an intestinal discomfort alleviating action.
(7) Use of dihomo-γ-linolenic acid for improving intestinal function through regulation of aquaporin 3 expression.
(8) Use of dihomo-γ-linolenic acid for alleviating intestinal discomfort through regulation of aquaporin 3 expression.
(9) A method of improving intestinal function through regulation of aquaporin 3 expression, including administering dihomo-γ-linolenic acid.
(10) A method of alleviating intestinal discomfort through regulation of aquaporin 3 expression, including administering dihomo-γ-linolenic acid.

### - Advantageous Effects of Invention

The present invention can provide a composition for improving intestinal function through regulation of aquaporin 3 expression, and use thereof. The present invention can also provide a method of improving intestinal function through regulation of aquaporin 3 expression, and a method of alleviating intestinal discomfort through regulation of aquaporin 3 expression.

### BRIEF DESCRIPTION OF DRAWINGS

Fig. 1 is a graph showing the proportion (%) of DGLA in fatty acids in phospholipids in the large intestine.
Fig. 2 is a graph showing results of analysis of aquaporin 3 gene expression.
Fig. 3 is a graph showing results of analysis of aquaporin 4 gene expression.

### DESCRIPTION OF EMBODIMENTS

### <Composition for improving intestinal function through regulation of aquaporin 3 expression>

DGLA may be derived from any suitable source. However, there are few known natural lipid sources having a high DGLA content, and minute amounts of DGLA may be extracted from bovine liver, porcine kidney, egg yolk, and the like. With the progress in the microbial fermentation technology in recent years, DGLA may be derived from microorganisms such as fungi, bacteria, or yeast.

Suitable fungi belong to the order of Mucorales, for example, *Mortierella, Pythium,* or *Entomophyhora.* Preferably, DGLA is derived from *Mortierella.* More preferably, DGLA is derived from *Mortierella alpina.* As disclosed in Japanese Patent No. 3354581, a DGLA-containing lipid can be produced by the microbial fermentation method using *Mortierella.* This method can prepare triglyceride in which DGLA accounts for about 40% of its constituent fatty acids.

DGLA is present in the form of a glyceride, a phospholipid, a glycolipid, an alkyl ester, or a free fatty acid, for example. DGLA used in the present invention may be in any of these forms. The glyceride is a glyceride containing DGLA as a constituent fatty acid. The glyceride may be, for example, a triglyceride, a diglyceride, or a monoglyceride. Preferably, the glyceride is at least one of a triglyceride or a diglyceride. One example of the glyceride containing DGLA as a constituent fatty acid is a triglyceride in which DGLA accounts for preferably at least 30%, more preferably at least 35% of its constituent fatty acids.

The composition for improving intestinal function of the present invention may also supply one or more types of additional PUFA in addition to DGLA. Such additional PUFA may be another n-6 PUFA (e.g., linoleic acid (LA) or γ-linolenic acid (GLA)) or n-3 PUFA (e.g., EPA or DHA) in addition to DGLA.

A physiologically acceptable functional derivative of DGLA, which can be converted to DGLA for use in the present invention, can be in the form of a DGLA-containing triglyceride, a DGLA-containing diglyceride, a DGLA-containing monoglyceride, a DGLA-containing phospholipid, a DGLA-containing glycolipid, a free fatty acid, a fatty acid ester (e.g., methyl or ethyl ester), or a sterol ester.

Preferably, PUFA is present in an oil. It can be a pure oil, a processed oil (e.g., chemically and/or enzymatically treated oil), or a concentrated oil. These oils may contain 10 to 100% PUFA. When the oil is derived from a microorganism, the amount of desired PUFA, such as DGLA, may be 5% or more, preferably 10% or more, more preferably 25% or more. The oil may contain one or more types of PUFA within the concentration range of the above percentage. The oil may be a single oil derived from a single cell or microorganism, or may be a blended or mixed oil of two or more oils derived from different sources. The oil may contain one or more additives, such as an antioxidant (e.g., tocopherol, Vitamin E, tocotrienol, an ascorbic acid derivative, a palmitate or a palmitic acid ester, or astaxanthin), sesamin, or CoQ10.

Preferably, the "improving intestinal function" in the composition for improving intestinal function of the present invention means different from improving decreased intestinal function due to ulcerative colitis.

As described above, it has been revealed that a decreased expression level of aquaporin 3 in the large intestine inhibits moisture absorption and causes diarrhea. DGLA has an action to promote moisture absorption and prevent diarrhea by inhibiting a decrease in aquaporin 3 expression in the large intestine. It has been also revealed that a decreased expression level of aquaporin 3 in the large intestine results in decreased intestinal barrier. DGLA inhibits a decrease in intestinal barrier function and maintains the intestinal barrier function by inhibiting a decrease in aquaporin 3 expression. Thus, DGLA improves the intestinal function by regulating aquaporin 3 expression. In the present invention, the intestinal function improvement may be promotion of moisture absorption or inhibition of decrease in moisture absorption in the large intestine. In the present invention, the composition for improving intestinal function through regulation of aquaporin 3 expression is preferably a composition for improving intestinal function through inhibition of decrease in aquaporin 3 expression, more preferably a composition for improving intestinal function through inhibition of decrease in aquaporin 3 expression in the intestine (preferably the large intestine). Preferably, a decrease in aquaporin 3 expression in the intestine does not include a decrease in aquaporin 3 expression due to ulcerative colitis.

Preferably, the composition for improving intestinal function through inhibition of decrease in aquaporin 3 expression is one for improving intestinal function through inhibition of aging-induced decrease in aquaporin 3 expression.

Preferably, the present invention is used by a human with decreased intestinal function, particularly a human with decreased intestinal function due to aging. The composition for improving intestinal function of the present invention can be used for prevention or amelioration of a condition or disease associated with a decrease in intestinal function due to a decrease in aquaporin 3 expression in the large intestine. Examples of the condition or disease associated with a decrease in intestinal function include constipation, diarrhea, and intestinal discomfort associated with constipation or diarrhea.

The composition for improving intestinal function of the present invention is applicable for both therapeutic use (medical use) and non-therapeutic use (non-medical use).

The composition for improving intestinal function of the present invention can be provided in the form of a food or beverage, a pharmaceutical product, a quasi-pharmaceutical product, feed, or the like. The composition for improving intestinal function of the present invention may be a food or beverage, a pharmaceutical product, a quasi-pharmaceutical product, feed, or the like for improving intestinal function by itself, or may be a material or formulation to be added to these products.

For example, the composition for improving intestinal function of the present invention can be provided in the form of an agent, but it is not limited thereto. Such an agent may be provided as a composition by itself or as a composition containing the agent. The composition for improving intestinal function of the present invention can also be referred to as an "intestinal function improving agent".

In order to sufficiently obtain the effect of the present invention, preferably, the composition for improving intestinal function of the present invention is an oral composition. The oral composition may be a food or beverage, an oral pharmaceutical product, or a quasi-pharmaceutical product, preferably a food or beverage or an oral pharmaceutical product, more preferably a food or beverage.

The composition for improving intestinal function of the present invention can contain optional additives and optional ingredients in addition to dihomo-γ-linolenic acid (DGLA), as long as the effect of the present invention is not impaired. Such additives and ingredients may be selected depending on the form of the composition, for example, and those generally usable in food and beverages, pharmaceutical products, quasi-pharmaceutical products, feed, and the like can be used.

When the composition for improving intestinal function of the present invention is provided as a food or beverage, a pharmaceutical product, a quasi-pharmaceutical product, feed, or the like, any common method can be used for the production.

For example, when the composition for improving intestinal function of the present invention is provided as a food or beverage, ingredients usable in food and beverages (e.g., food materials, and food additives that are used as needed) are added to dihomo-γ-linolenic acid (DGLA) to provide various food and beverages. The food or beverage is not limited. Examples thereof include general food and beverages, health foods, dietary supplements, health drinks, foods with function claims, foods for specified health uses, and foods for the sick. The health foods, dietary supplements, foods with function claims, foods for specified health uses, and the like can be provided, for example, in various formulation forms such as fine granules, tablets, granules, powders, capsules, chewable tablets, syrups, liquid agents, and liquid foods.

The composition for improving intestinal function of the present invention can also be provided in the form of a food or beverage labeled as having an intestinal function improving effect based on regulation of aquaporin 3 expression.

The composition for improving intestinal function of the present invention may be labeled as having an intestinal discomfort alleviating action. The composition for improving intestinal function of the present invention may also be labeled as having an intestinal function improving effect. Preferably, such labels include, for example, one or two or more selected from the group consisting of "alleviating intestinal discomfort", "regulating the intestine", "clearing the intestine", "improving fecal conditions such as constipation and diarrhea", "improving bowel movement", "improving bowel movement habit", "improving intestinal conditions", "enhancing intestinal barrier function", "maintaining intestinal function", and "improving decreased intestinal function".

Preferably, the composition for improving intestinal function of the present invention is used for one or two or more purposes selected from the group consisting of alleviating intestinal discomfort, regulating the intestine, clearing the intestine, improving fecal conditions such as constipation and diarrhea, improving bowel movement, improving bowel movement habit, improving intestinal conditions, enhancing intestinal barrier function, maintaining intestinal function, and improving decreased intestinal function. In particular, use for alleviating intestinal discomfort is preferred.

The dihomo-γ-linolenic acid (DGLA) content of the composition for improving intestinal function of the present invention is not limited, and can be set according to the form of the composition, for example. Preferably, the dihomo-γ-linolenic acid (DGLA) content of the composition for improving intestinal function is an amount suitable for ingestion of 5 mg to 600 mg of dihomo-γ-linolenic acid (DGLA) (free DGLA equivalent) per day per adult, for example. For example, the intake of DGLA (free DGLA equivalent) may be 5 mg to 200 mg, or 5 mg to 150 mg per day per adult. In one embodiment, when the composition for improving intestinal function of the present invention is provided as a food or beverage, a pharmaceutical product, a quasi-pharmaceutical product, feed, or the like, preferably, the DGLA content of the food or beverage, the pharmaceutical product, the quasi-pharmaceutical product, the feed, or the like is in the above range.

The composition for improving intestinal function of the present invention can be ingested by or administered to a subject by a suitable method depending on the form. As described above, in order to sufficiently obtain the effect of the present invention, oral ingestion (oral administration) of the composition for improving intestinal function of the present invention is preferred.

The intake (which can also be referred to as "dosage") of the composition for improving intestinal function of the present invention is not limited, and may be an amount (effective amount) that achieves the intestinal function improving effect. The intake may be appropriately set according to the administration form, administration method, body weight of a subject, and the like. In one embodiment, when the composition for improving intestinal function is orally ingested by or administered to a human (adult), the intake of the composition in terms of the intake of dihomo-γ-linolenic acid (DGLA) (free DGLA equivalent) is preferably 5 to 600 mg, more preferably 5 to 200 mg per day per 60 kg body weight. Ingestion of the composition in the above amount in at least one portion a day, for example, one to several (e.g., two or three) portions a day, is preferred. In one embodiment, the composition for improving intestinal function of the present invention may be an oral composition for allowing the above amount of dihomo-γ-linolenic acid (DGLA) to be ingested by or administered to an adult (60 kg body weight) per day.

Continuous ingestion (administration) of dihomo-γ-linolenic acid (DGLA) enhances the intestinal function improving effect. Thus, in a preferred embodiment, the composition for improving intestinal function of the present invention is continuously ingested. In one embodiment of the present invention, the composition for improving intestinal function is continuously ingested preferably for two weeks or more, more preferably for 12 weeks or more.

The composition for improving intestinal function of the present invention may be ingested by or administered to any subject (which can also be referred to as an "administration subject"). The subject is preferably a human or non-human mammal, more preferably a human.

Preferably, the subject of ingestion of the composition for improving intestinal function of the present invention is one needing or wanting intestinal function improvement. Examples of such a subject include a human with constipation, diarrhea, or intestinal discomfort due to constipation or diarrhea. The composition for improving intestinal function of the present invention can also be used by a healthy person, for example, for the purpose of preventing a condition or disease that is likely to be prevented or alleviated by improving intestinal function.

The composition for improving intestinal function of the present invention contains, as an active ingredient, a highly safe component (DGLA) present in natural products and food and beverages. Continuous ingestion of the composition achieves the intestinal function improving effect at high levels. Thus, the composition for improving intestinal function of the present invention is useful as a health food, a food with function claims, or the like for regulating aquaporin 3 expression in the intestine (e.g., inhibiting a decrease in expression) and thus improving the intestinal function of a person with the conditions described above (constipation, diarrhea, and intestinal discomfort due to constipation or diarrhea), a healthy person, or the like through daily oral ingestion of the composition.

Preferably, the present invention is used by a subject with decreased intestinal function for purposes such as preventing or ameliorating a condition or disease resulting from or involving a decrease in intestinal function and preserving and maintaining health by improving the intestinal function through regulation of aquaporin 3 expression in the large intestine by ingestion of dihomo-γ-linolenic acid (DGLA).

While the composition for improving intestinal function of the present invention and the use thereof are useful in improving a function that regulates moisture absorption in the small or large intestine through regulation of aquaporin 3 expression, the composition and the use thereof are useful in promoting moisture absorption in the small or large intestine, and are particularly useful in promoting moisture absorption in the large intestine.

The present invention also encompasses a method of improving intestinal function, including administering dihomo-γ-linolenic acid (DGLA). Preferably, the method of improving intestinal function of the present invention is performed by orally administering a composition for improving intestinal function containing DGLA.

The present invention also relates to use of dihomo-γ-linolenic acid for improving intestinal function through regulation of aquaporin 3 expression. Preferably, the use is use of dihomo-γ-linolenic acid by oral ingestion.

The present invention also relates to use of dihomo-γ-linolenic acid for alleviating intestinal discomfort through regulation of aquaporin 3 expression. Preferably, the use is use of dihomo-γ-linolenic acid by oral ingestion.

The present invention also relates to a method of improving intestinal function through regulation of aquaporin 3 expression, including administering dihomo-γ-linolenic acid. Preferably, the method is oral administration of dihomo-γ-linolenic acid.

The present invention also relates to a method of alleviating intestinal discomfort through regulation of aquaporin 3 expression, including administering dihomo-γ-linolenic acid. Preferably, the method is oral administration of dihomo-γ-linolenic acid.

The composition for improving intestinal function of the present invention and the use thereof may be a composition for improving small intestinal or large intestinal function and use thereof, preferably a composition for improving large intestinal function and use thereof. The method of improving intestinal function and the method of alleviating intestinal discomfort of the present invention may be methods that include administering the composition for improving small intestinal or large intestinal function, and are preferably methods that include administering the composition for improving large intestinal function.

### EXAMPLES

The present invention is described in more detail below with reference to examples, but the present invention is not limited to these examples. In the present invention,"%" means "wt%" unless otherwise stated.

### <Example 1>

In order to examine whether ingestion of DGLA has any action on the intestinal function that decreases due to aging, triglyceride in which DGLA accounts for about 40% of its constituent fatty acids (i.e., a DGLA lipid), which was prepared by the method disclosed in Japanese Patent No. 3354581, was used to examine its usefulness in experimental animals. The animal experiments were performed based on a plan approved by the relevant chief through evaluation of the in-house animal experiment committee, in compliance with the animal welfare management laws and other related laws and regulations.

In the experiment, young (3 months) and old (17 to 26 months) mice (male C57BL/6J) were used. The group composition was as follows: a young control diet group (10 mice), an old control diet group (14 mice), and an old DGLA diet group (12 mice). The following two types of feed shown in Table 1 were prepared. The young control diet group and the old control diet group were allowed free access to control diet for 12 weeks. The old DGLA diet group was allowed free access to DGLA diet for 12 weeks. The DGLA diet was prepared by adding the triglyceride (0.5%) to feed such that the amount of DGLA in the feed was about 0.2% (calculated as the amount of free fatty acids). The control diet and the DGLA diet were prepared such that the proportions of fatty acids excluding DGLA were almost the same between these diets. In each of the young control diet group, the old control diet group, and the old DGLA diet group, the mean daily intake was about 3 g. Since the mean body weight of the mice was about 30 g, the DGLA intake in this experiment was estimated to be about 200 mg/kg per day.

The animals were euthanized under anesthesia at the end of the experiment after 12 weeks of ingestion, and large intestine was collected. The large intestine was then subjected to fatty acid analysis and gene expression evaluation.

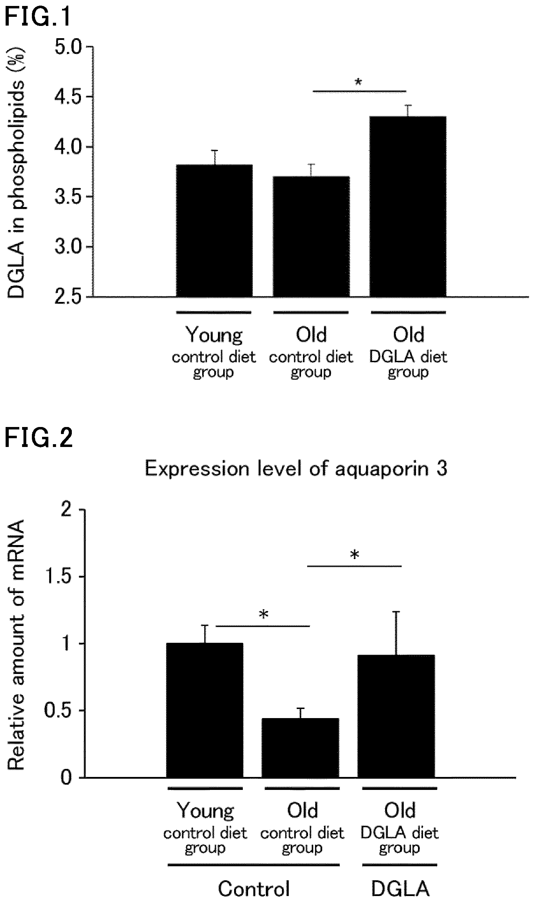

The total lipid in the tissues was extracted and purified from the collected large intestine by the Folch method. Subsequently, a phospholipid fraction was obtained using thin layer chromatography (hexane:diethyl ether = 7:3), and the fraction was methyl-esterified with hydrochloric acid methanol and subjected to gas chromatography (Agilent 7890B) analysis. The proportion (%) of DGLA in fatty acids in phospholipids in the large intestine was calculated based on the obtained peak area. Fig. 1 shows the results.

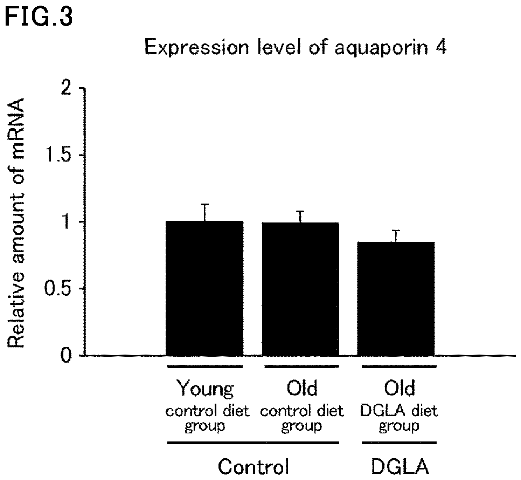

The aquaporin gene expression level in the large intestine was measured. The total RNA was extracted from the frozen large intestine using ISOGEN (Nippon Gene Co., Ltd.). The resulting total RNA was reverse-transcribed using a High Capacity cDNA Reverse Transcription Kit (Thermo Fisher Scientific). Quantitative PCR was performed in a StepOnePlus^{®} real-time PCR system (Thermo Fisher Scientific) using gene-specific primers and TaqMan^{®} Fast Universal PCR Master Mix (Thermo Fisher Scientific). Fig. 2 and Fig. 3 show the results. The results were calibrated using the expression level of 18S, and expressed in an appropriate unit. Table 3 shows the gene-specific primers used for detection.

### Table 1: Table of ingredients in each type of feed (unit: g)

**[Table 1]**

| Name of raw material (per 1000 g) | Control diet | DGLA diet |
|---|---|---|
| Casein | 140.00 | 140.00 |
| L-cystine | 1.80 | 1.80 |
| Cornstarch | 465.69 | 465.69 |
| α-Cornstarch | 155.00 | 155.00 |
| Sucrose | 100.00 | 100.00 |
| Cellulose powder | 50.00 | 50.00 |
| AIN-93M mineral mix | 35.00 | 35.00 |
| AIN-93 vitamin mix | 10.00 | 10.00 |
| Choline bitartrate | 2.50 | 2.50 |
| Tertiary butylhydroquinone | 0.01 | 0.01 |
| Palm oil | 21.14 | 21.00 |
| Soybean oil | 11.43 | 6.00 |
| Linseed oil | 7.43 | 8.00 |
| DGLA-TG (*1) | 0.00 | 5.00 (*2) |
| Total | 1000.00 | 1000.00 |

| | | |
|---|---|---|
| *1: Triglyceride in which DGLA accounts for about 40% of its constituent fatty acids *2: Corresponding to about 0.2% in the feed (free DGLA equivalent) | | |

### Table 2: Body weight transition (unit: g, mean ± standard error)

**[Table 2]**

| | At the start of ingestion | Week 4 of ingestion | Week 8 of ingestion | Week 12 of ingestion |
|---|---|---|---|---|
| Young control diet group (N = 10) | 24.8 ± 0.3 | 26.4 ± 0.3 | 28.7 ± 0.7 | 31.2 ± 1.0 |
| Old control diet group (N = 14) | 34.3 ± 0.8 | 33.2 ± 1.0 | 32.4 ± 1.1 | 31.5 ± 0.9 |
| Old DGLA diet group (N = 12) | 36.1 ± 0.8 | 35.7 ± 1.2 | 34.6 ± 0.8 | 32.6 ± 0.5 |

**[Table 3]**

| Gene name | Entrez Gene ID | Manufacturer | Primer |
|---|---|---|---|
| Aqp3 | 11828 | Thermo Fisher Scientific | Mm01208559_m1 |
| Aqp4 | 11829 | Thermo Fisher Scientific | Mm00802131_m1 |

### (Results)

The old DGLA diet group showed no abnormality in the body weight transition (Table 2) or general conditions, compared to the old control diet group.

Fig. 2 shows the proportion (%) of DGLA in fatty acids in phospholipids in the large intestine (mean ± standard error). Significance was determined by Dunnett's Test (vs. old control diet group, *: p < 0.05). Ingestion of DGLA diet increased the proportion of DGLA in phospholipids in the large intestine (young control diet group: 3.82 ± 0.14%; old control diet group: 3.71 ± 0.13%; and old DGLA diet group: 4.30 ± 0.11%). The above shows that ingestion of DGLA increases the proportion of DGLA in fatty acids constituting phospholipids in the large intestine.

Fig. 2 and Fig. 3 show the results of aquaporin gene expression analysis in the large intestine.

Fig. 2 shows the results of analysis of aquaporin 3 gene (Aqp3) expression (mean ± standard error, *: p < 0.05 (vs. old control diet group)). The graph confirmed that DGLA inhibited an aging-induced decrease in aquaporin 3 expression. Fig. 3 shows the results of analysis of aquaporin 4 gene (Aqp4) expression (mean ± standard error). The graph confirmed that DGLA had no effect on the expression level of aquaporin 4.

The above confirmed that DGLA inhibits aging-induced decrease in aquaporin 3 expression in the large intestine. Specifically, DGLA was confirmed to be effective in improving the intestinal function through regulation of aquaporin 3 expression.

## Claims

1. A composition for improving intestinal function through regulation of aquaporin 3 expression, comprising:
dihomo-γ-linolenic acid as an active ingredient.

2. The composition for improving intestinal function according to claim 1,
wherein the regulation of aquaporin 3 expression is inhibition of decrease in aquaporin 3 expression.

3. The composition for improving intestinal function according to claim 2,
wherein the inhibition of decrease in aquaporin 3 expression is inhibition of aging-induced decrease in aquaporin 3 expression.

4. The composition for improving intestinal function according to any one of claims 1 to 3,
wherein the composition for improving intestinal function is a food or beverage, a pharmaceutical product, or a quasi-pharmaceutical product.

5. The composition for improving intestinal function according to any one of claims 1 to 4,
wherein the composition is used for one or two or more purposes selected from the group consisting of alleviating intestinal discomfort, regulating the intestine, clearing the intestine, improving fecal conditions, improving bowel movement, improving bowel movement habit, improving intestinal conditions, enhancing intestinal barrier function, maintaining intestinal function, and improving decreased intestinal function.

6. The composition for improving intestinal function according to any one of claims 1 to 5,
wherein the composition is labeled as having an intestinal discomfort alleviating action.

7. Use of dihomo-γ-linolenic acid for improving intestinal function through regulation of aquaporin 3 expression.

8. Use of dihomo-γ-linolenic acid for alleviating intestinal discomfort through regulation of aquaporin 3 expression.

9. A method of improving intestinal function through regulation of aquaporin 3 expression, comprising:
administering dihomo-γ-linolenic acid.

10. A method of alleviating intestinal discomfort through regulation of aquaporin 3 expression, comprising:
administering dihomo-γ-linolenic acid.
